# EUROPEAN PATENT SPECIFICATION

(11) **EP 3 713 433 B1**
(45) Date of publication and mention of the grant of the patent: **12.01.2022**
(21) Application number: 18826187.9
(22) Date of filing: 23.11.2018
(51) Int. Cl.: A24F 40/30, A24F 40/485

(54) **APPARATUS, SYSTEM AND METHOD FOR GENERATING AN INHALABLE MEDIUM**
VORRICHTUNG, SYSTEM UND VERFAHREN ZUR ERZEUGUNG EINES INHALIERBAREN MEDIUMS
APPAREIL, SYSTÈME ET PROCÉDÉ DE GÉNÉRATION D'UN MILIEU INHALABLE

(30) Priority: 24.11.2017 GB 201719578
(43) Date of publication of application: 30.09.2020
(73) Proprietor: Nicoventures Trading Limited, London WC2R 3LA (GB)
(72) Inventor: AZZOPARDI, Anna, London Greater London WC2R 3LA (GB); SPENCER, Alfred, London Greater London WC2R 3LA (GB)
(74) Representative: Dehns
(86) International application number: PCT/EP2018/082411
(87) International publication number: WO 2019/101946

(56) References cited:
- WO-A1-2013/050934
- WO-A1-2015/179388
- WO-A2-2017/194763
- CN-A- 106 037 011

## Description

### Technical Field

The present invention relates to apparatus and system for generating an inhalable medium, and to a method of generating an inhalable medium.

### Background

Smoking articles such as cigarettes, cigars and the like burn tobacco during use to create tobacco smoke. Attempts have been made to provide alternatives to these articles that burn tobacco by creating products that release compounds without burning. Examples of such products are heating devices which release compounds by heating, but not burning, the material. The material may be for example tobacco or other non-tobacco products, which may or may not contain nicotine.

CN106037011 describes an apparatus having a first and second chamber in communication with each other, each chamber comprising a heat generating component. External airflow sequentially passes through the first and second chamber.

### Summary

According to a first aspect of the present invention, there is provided an apparatus as claimed in claim 1.

In an example, one of the first and second receptacles is configured to receive a consumable product of a predetermined size, the consumable product comprising one of the first and second substrates, and wherein the other of the first and second receptacles is configured to directly receive the other of the first and second substrates. For example, the first receptacle may be configured to directly receive the first substrate and the second receptacle may be configured to receive a consumable comprising the second substrate, the consumable having a predetermined size.

In an example, a receptacle configured to directly receive a substrate comprises a chamber having an opening to provide access to the chamber; and a member movable to cover or uncover the opening. For example the receptacle may comprise a chamber and a lid to cover an aperture formed in the chamber, such that in use the substrate is directly received in the chamber via the aperture.

In another example, the first receptacle is configured to receive a consumable product comprising the first substrate and having a first predetermined size and the second receptacle is configured to receive a second consumable product comprising the second substrate and having a second predetermined size.

The first and second flow paths may define separate paths, or channels, through which the heated products or inhalable medium may travel before being inhaled by a user.

In an example, the apparatus comprises a flow path selection assembly movable between at least a first position in which the first flow path is operative, and a second position in which the second flow path is operative. Moving between at least the first position and the second position may be moving from the first position to the second position, or from the second position to the first position.

In an example, the flow path selection assembly comprises a control member, the control member being actuatable by a user, wherein actuation of the control member causes the flow path selection assembly to move between at least the first and second positions.

In an example, the housing comprises a first outlet and a second outlet, wherein the first flow path includes the first outlet, and the second flow path includes the second outlet.

In the example comprising the first and second outlets, each of the first and second flow paths has its own separate outlet.

In an example, the apparatus comprises a removeable mouthpiece, the removeable mouthpiece attachable to both the first and second outlets. For example, when the apparatus comprises first and second flow paths comprising first and second outlets respectively, the removeable mouthpiece may be attachable to either the first or second outlets.

In an alternative example, the housing comprises an outlet, and wherein both the first flow path and the second flow path include the outlet.

According to a second aspect of the present invention there is provided a system as claimed in claim 9.

In an example, the system further comprises a removeable mouthpiece.

According to a third aspect of the present invention there is provided a method as claimed in claim claim 10.

In an example, the method further comprises heating the second solid substrate using the second heater. For example, heating may occur before, during, and/or after entraining the one or more constituents from the second substrate in the heated products.

In an example, the method further comprises:
receiving a consumable product of a predetermined size in one of the first and second receptacles, the consumable product comprising one of the first and second substrates; and
directly receiving the other of the first and second substrates in the other of the first and second receptacles.

In an example, directly receiving one of the first and second substrates in the other of the first and second receptacles comprises moving a member to uncover an opening in a chamber of the other of the first and second receptacles, for example opening a lid in a chamber of the other of the first and second receptacles. In one example, opening the lid reveals an aperture formed in the chamber.

In an example, the method further comprises selecting one of the first and second flow paths as an operative flow path.

In an example, selecting one of the first and second flow paths comprises moving a selection assembly between at least a first position, in which the first flow path is the operative flow path, and a second position in which the second flow path is the operative flow path.

In an example, moving the selection assembly comprises actuating a control member.

In an example, the method further comprises at least one of:
passing the inhalable medium along the first flow path and through a first outlet in the housing; and
passing the inhalable medium along the second flow path and through a second outlet in the housing.

In an example, the method further comprises at least one of:
passing the inhalable medium along the first flow path and through an outlet in the housing; and
passing the inhalable medium along the second flow path and through the outlet in the housing.

### Brief Description of the Drawings

Embodiments of the invention will now be described, by way of example only, with reference to the accompanying drawings, in which:
Figure 1 shows a schematic partial cross-sectional view of an example of an apparatus for generating an inhalable medium not covered by the present claims;
Figure 2 shows a schematic partial cross-sectional view of another example of an apparatus for generating an inhalable medium not covered by the present claims;
Figure 3 shows a schematic partial cross-sectional view of another example of an apparatus for generating an inhalable medium not covered by the present claims, the example apparatus comprising a mouthpiece;
Figure 4 shows a schematic partial cross-sectional view of another example of an apparatus for generating an inhalable medium, the example apparatus comprising first and second flow paths and first and second outlets;
Figure 5 shows a schematic partial cross-sectional view of another example of an apparatus for generating an inhalable medium, the example apparatus comprising first and second flow paths, first and second outlets, and a mouthpiece;
Figure 6 shows a schematic partial cross-sectional view of another example of an apparatus for generating an inhalable medium, the example apparatus comprising first and second flow paths and a single outlet;
Figure 7A shows a schematic partial cross-sectional view of another example of an apparatus for generating an inhalable medium, the example apparatus comprising a first flow path as being operative, and a flow path selection assembly arranged in a first position;
Figure 7B shows a schematic partial cross-sectional view of another example of an apparatus for generating an inhalable medium, the example apparatus comprising a second flow path as being operative, and a flow path selection assembly arranged in a second position; and
Figure 8 shows an example method of generating an inhalable medium according to an example not covered by the present claims.

### Detailed Description

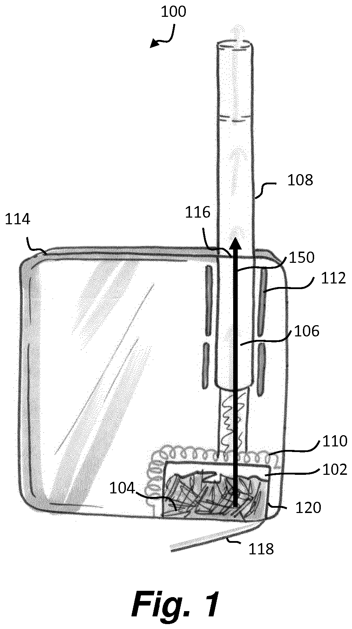

Referring to Figure 1, there is shown an example of an apparatus 100 for generating an inhalable medium. In broad outline, a first heater 110 within the apparatus 100 heats a first substrate 104 in a first receptacle 102 to form heated products, which pass through a second receptacle 106, in which a second substrate 108 is located, so as to produce an inhalable medium that contains one or more constituents derived from the first and second substrates 104, 108. A second heater 112 may also be provided to heat the second substrate 108.

The apparatus therefore allows the inhalable medium to have, for example, a flavour or flavours that are derived from both the first and second substrates 104, 108 contained in the apparatus in use. For example, such an apparatus 100 allows a user of the apparatus to create and control the flavour of the inhalable medium by selecting specific combinations of solid and/or gel substrates.

In one example, the heated products from the first substrate pass over, or through, the second substrate or combine with heated products from the second substrate to produce the flavoured inhalable medium. The heated products from the first substrate are hot and so heat, or additionally heat, the second substrate. The use of two heaters 110, 112 may allow a lower temperature to be used for the second heater, or allow the second heater to operate for less time, because the heated products from the first substrate contribute to the heating of the second substrate.

The first and second substrates may be solids or gels. In one example, a solid substrate may consist of, or comprise tobacco. Tobacco products may include loose leaf tobacco, or a consumable product comprising tobacco. A consumable product may be, for example, of a predetermined or specific size that is configured to be placed within a receptacle sized to receive the consumable product. Consumable products of a predetermined size may have a specific volume, or have specific dimensions suitable for use with the apparatus. In one example, a consumable product is tubular in nature, and may be known as a "tobacco stick". In some examples the consumable product may comprise tobacco formed in a specific shape which is then coated, or wrapped in one or more other materials, such as paper or foil.

In another example, a solid substrate may be a flavour pouch, designed to impart flavour upon application of heat by a heater and/or heated products passing through, and/or over the flavour pouch. In some examples, the flavour pouch may also comprise tobacco and/or nicotine. Flavour pouches may be sold in predetermined sizes, and may also be known as consumable products. Flavour pouches may comprise flavour producing material inside a porous material, for example.

A gel may be a solid, jelly like material. The gel may be a Newtonian or non-Newtonian gel. In one example, the gel is a thermoplastic gel. In one example, the gel has a viscosity of between 0.1 and 100 Ns/m². In contrast, an e-liquid may have a lower viscosity, for example a viscosity of less than 0.1 Ns/m², for example between 0.010 and 0.099 Ns/m². Example values for the e-liquid include around 0.02 and around 0.096 Ns/m², for example. In one example, a gel substrate may include nicotine, and/or one or more flavourants. In one particular example, the gel substrate is in the form of a sheet. The gel substrate may be designed to release or impart nicotine and/or flavour upon application of heat by a heater and/or heated products passing through, or over the gel substrate. Gel substrates may also be sold in predetermined sizes, and may also be known as consumable products.

In Figure 1, the apparatus 100 of this example comprises a housing 114. The housing 114 has an open end which defines an outlet 116. In this example, the second substrate 108 is a solid consumable product, that is partially inserted into the outlet 116 so that it is received within the second receptacle 106. In this example, the first substrate 104 is loose leaf tobacco, and can be placed within the first receptacle 102 by a user, for example via an opening in the housing 114 (not shown).

The first heater 110 is used to heat the first substrate 104, and the second heater 112 may be used to heat the second substrate 108. The heaters are therefore arranged within the housing to allow heating of the substrates. In this example, the heaters 110, 112 are powered by a battery (not shown) and are electrically connected to the battery. The heaters 110, 112 may be electrically resistive heaters, including for example a nichrome resistive heater, a ceramic heater, etc. A heater 110, 112 may be for example a wire, which may for example be in the form of a coil, a plate (which may be a multi-layer plate of two or more different materials, one or more of which may be electrically conductive and one or more of which may be electrically non-conductive), a mesh (which may be woven or non-woven for example, and which again may be similarly multi-layer), a film heater, etc. Other heating arrangements may be used, including non-electrical heating arrangements. In the example of Figure 1, the first heater 110 is in the form of a coil, and the second heater 112, is an oven heater in thermal contact with the second substrate 108 to pre-heat the second substrate 108 and/or provide additional heat to the second substrate 108. This encourages release of constituents from the second substrate 108 as the heated products from the first substrate 104 passes through the second substrate 108 in use.

The first and second receptacles 102, 106 defined within the housing 114 hold or contain the first and second substrates 104, 108 respectively. In use, a user can access the receptacles 102, 106 to replace or replenish the substrate 104, 108. Various different forms for the receptacles 102, 106 may be used. For example, the receptacles 102, 106 may be a tube which is completely open at both ends and which contains the substrate 104, 108. As another example, the receptacles 102, 106 may be a tube or container which has one or more end walls which have through holes through which heated products can pass. The receptacles 102, 106 may remain in situ within the housing 114 whilst the user removes and replaces the substrate 104, 108. Alternatively, a receptacle 102, 106 may be a discrete item which in use is inserted into and removed from the housing 114 as a whole.

In one example, a receptacle is configured to directly receive a substrate. Directly receiving a substrate in the receptacle allows a quantity, or volume of substrate to be selected by a user, for example the substrate may not have a predetermined size, or may have an indeterminate size. An example of such a directly received substrate includes loose material, for example a loose particulate material such as loose leaf tobacco, where the user is free to place any desired quantity of the loose material into the receptacle. When the receptacle receives a quantity, or volume of substrate selected by a user, the receptacle, or apparatus may be known as an open system. Conversely, when the receptacle receives a consumable product of a predetermined size, the receptacle, or apparatus may be known as a closed system. Accordingly, one of the first and second receptacles may be a closed system, and the other of the first and second receptacles may be an open system. The example apparatus may therefore be known as an open and closed hybrid apparatus, or device. The user is therefore provided with an apparatus that is adaptable to specific needs. The user may purchase standard consumable products of a predetermined size, and control and combine flavours with a quantity of substrate they desire, to suit their preferences.

An example of such a directly received substrate includes loose leaf tobacco, where the user is free to place any desired quantity of loose leaf tobacco into the receptacle. Such a receptacle may therefore be configured to receive a loose material. The receptacle 102 may comprise a chamber 120 (such as a box or container) and a member or lid 118 to cover an opening or aperture formed in the chamber 120. The substrate 104 can be replenished by opening the lid 118 so that the aperture is uncovered, and the substrate 104 can be directly received in the chamber 120 via the aperture. The lid 118 depicted in Figure 1 is hinged to allow the chamber 120 to be accessed, but may be movable in any other suitable manner to cover and uncover the aperture, for example it may be slideable. In another example, the receptacle comprises a chamber with an aperture, and it is the housing 114 that comprises a lid. Opening the lid in the housing 114 allows the chamber to be accessed. This construction allows a user to easily access the receptacle to add or remove the substrate. For example, the user moves the member or opens the lid, and adds or removes the substrate from the chamber. In one example, a loose substrate is received in the chamber in this manner.

Although Figure 1 depicts two solid substrates, the substrates may be solid or gel. The first receptacle 102 is therefore configured to receive a solid substrate or a gel substrate. Accordingly, the first receptacle 102 is not configured to receive a liquid. Similarly, the second receptacle 106 is configured to receive a solid substrate or a gel substrate. Accordingly, the second receptacle 106 is not configured to receive a liquid.

The housing 114 defines a flow path, illustrated by arrow 150. The flow path includes the first and second receptacles 102, 106, such that in use heated products produced by heating the first substrate 104 received in the first receptacle 102 pass through the second receptacle 106 to produce the inhalable medium, which exits the housing 114 through the outlet 116. In the example of Figure 1, the inhalable medium travels through the outlet 116, whilst being drawn through the second substrate 108.

The flow path may also comprise one or more air inlets (not shown), through which air passes into the apparatus 100, before entering the first receptacle 102.

In particular examples, substrates 104, 108 are porous so that the heated products (such as heated gases) pass through the substrates 104, 108. For example, heated products produced by the first substrate may pass through the second substrate 108, before passing through the outlet 116. In other example, the substrates 104, 108 are not porous, or are only partially porous, and heated products produced by the substrate 104, 108 mix and combine with other heated products to produce the inhalable medium.

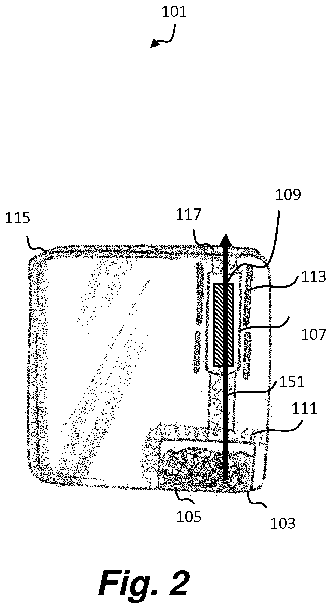

Referring now to Figure 2, there is shown another example of an apparatus 101 for generating an inhalable medium. It will be understood that the arrangements and alternatives, etc. described above in relation to the example of Figure 1 are also applicable to the example of Figure 2, as well as other examples described herein.

Figure 2, shows an example of another apparatus 101 for generating an inhalable medium. In broad outline, a first heater 111 within the apparatus 101 heats a first substrate 105 in a first receptacle 103 to form heated products, which pass through a second receptacle 107, in which a second substrate 109 is located, so as to produce an inhalable medium that contains one or more constituents derived from the first and second substrates 105, 109. A second heater 113 may also heat the second substrate 109.

In Figure 2, the apparatus 101 of this example comprises a housing 115. The housing 115 has an open end which defines an outlet 117. In this example, as in Figure 1, the second substrate 109 is a solid consumable product, however, unlike in Figure 1, the consumable product is fully inserted into the housing 115 so that it is received within the second receptacle 107. The second substrate 109 may be received in the second receptacle 107 via an opening in the housing 115 (not shown), or via the outlet 117.

The first heater 111 is used to heat the first substrate 105, and the second heater 113 may be used to heat the second substrate 108.

The housing 115 defines a flow path, illustrated by arrow 151. The flow path includes the first and second receptacles 103, 107, such that in use heated products produced by heating the first substrate 105 received in the first receptacle 103 pass through the second receptacle 107 to produce the inhalable medium, which exits the housing through the outlet 117. In the example of Figure 2, the inhalable medium travels directly through the outlet 117. The flow path may also comprise one or more air inlets (not shown), through which air passes into the apparatus 100, before entering the first receptacle 102.

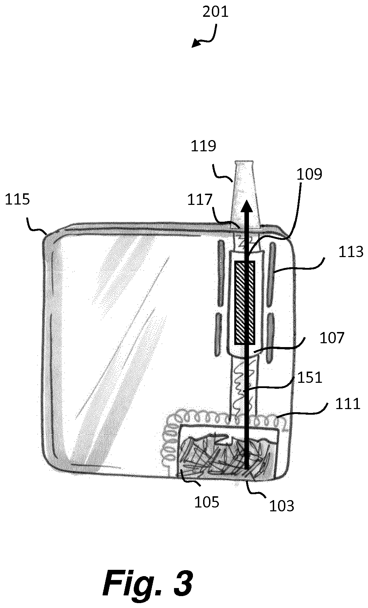

Figure 3 shows another example apparatus 201. In this example, the apparatus 201 is substantially the same as apparatus 101, however a mouthpiece 119 has been fitted onto the housing, adj acent to the outlet 117. The mouthpiece 119 provides a more ergonomic design for a user to inhale the inhalable medium. In this example, the mouthpiece 119 is removable by a user from the housing 115 and an O-ring or other seal assists in sealing the mouthpiece 119 in the housing 115. In other examples, the mouthpiece 119 is designed to be permanently attached to the housing 115.

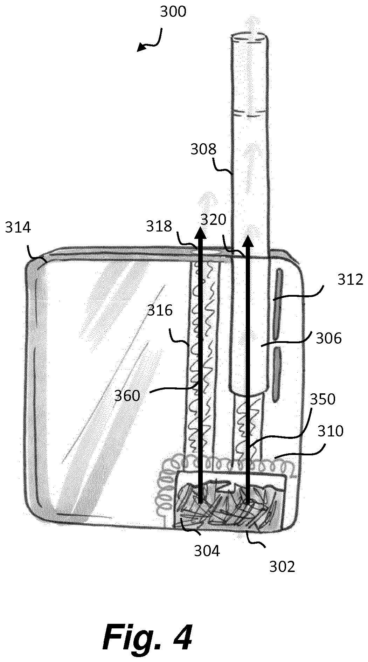

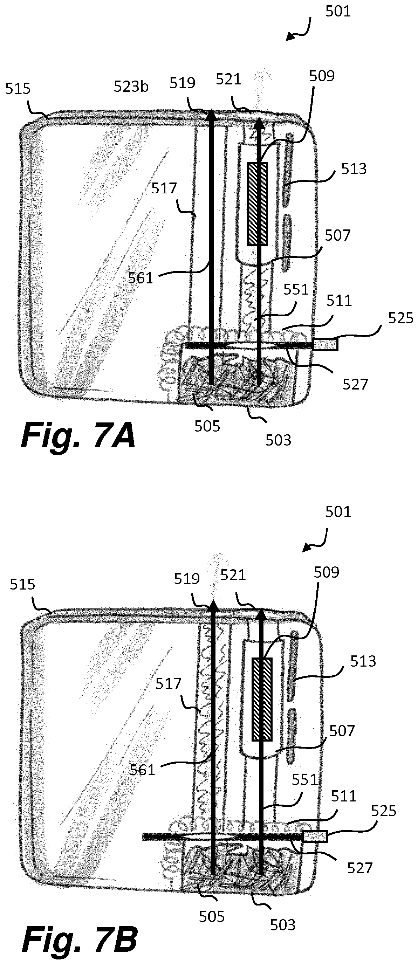

Figure 4 shows an apparatus 300 for generating an inhalable medium. In broad outline, the apparatus 300 comprises a housing 314 that defines a first flow path (illustrated by arrow 350), and a second flow path (illustrated by arrow 360). In use, only one of these flow paths may be operative at any one time, and a user may select, or choose which is the operative flow path. Selection of an operative flow path is discussed in more detail in relation to Figures 7A and 7B. In the example of Figure 4, the housing comprises a first outlet 320 and a separate second outlet 318.

Such a construction comprising first and second flow paths allows a user to inhale a medium produced by, and therefore flavoured by, the first substrate only, if desired. Alternatively, the user may choose to inhale a medium produced by, and therefore flavoured by, the second substrate only, if desired. Alternatively, the user may choose to inhale a medium produced by, and therefore flavoured by, the first and second substrates, if desired. The example apparatus therefore provides the user with greater choice during use of the apparatus. The second substrate may remain in the second receptacle while the second flow path is operative, such that the user need not remove the second substrate if they choose to heat only the first substrate. This may be particularly useful if the second substrate is spent, depleted or fully used, but has not yet been removed from the second receptacle for replenishment or disposal.

Figure 4 depicts a first heater 310 within the apparatus 300 to heat a first substrate 304 in a first receptacle 302 to form heated products. If the first flow path 350 is operative, the heated products produced in the first receptacle 302, will pass through a second receptacle 306, in which a second substrate 308 is located, so as to produce an inhalable medium that contains one or more constituents derived from the first and second substrates 304, 308. A second heater 312 may also heat the second substrate 308. Alternatively, if the second flow path 360 is operative, the heated products produced in the first receptacle 302, may pass through a channel, or conduit 316 but not pass through the second receptacle 306, so as to produce an inhalable medium that contains one or more constituents derived from the first substrate 304 only.

Accordingly, the first flow path 350 therefore includes the first receptacle 302 and the second receptacle 306. The second flow path 360 includes the first receptacle 302 but not the second receptacle 306. The second flow path may include the first receptacle 302 and the conduit 316 arranged outside of the first flow path 350. In the example of Figure 4, the first flow path 350 includes the first outlet 320 and the second flow path 360 includes the second outlet 318. Each flow path therefore may have its own separate outlet.

In the example of Figure 4, the second substrate 308 may need to be removed before a user inhales the inhalable medium. A mouthpiece (not shown) may be attachable to the second outlet 318.

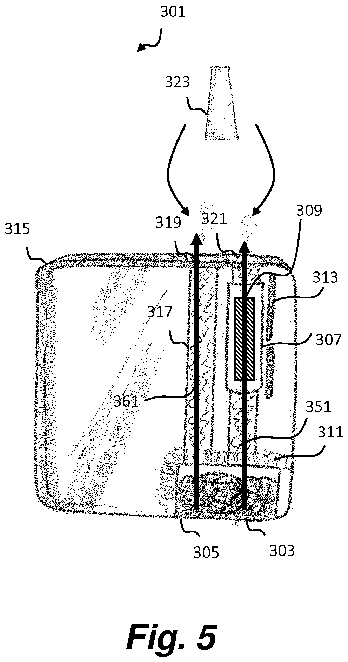

Figure 5 shows an alternative apparatus 301 for generating an inhalable medium. In broad outline, the apparatus 301 is substantially similar to the apparatus 300 of Figure 4, but instead of being configured to receive a second substrate of the form depicted in Figure 4, the housing 315, may be configured to fully receive the second substrate 309. Figure 5 comprises a housing 315, that defines a first flow path (illustrated by arrow 351) and a second flow path (illustrated by arrow 361). Again, in use, only one of these flow paths may be operative at any one time, and a user may select, or choose which is the operative flow path. In the example of Figure 5, the housing comprises a first outlet 321 and a second outlet 319.

Figure 5 depicts a first heater 311 within the apparatus 301 to heat a first substrate 305 in a first receptacle 303 to form heated products. If the first flow path 351 is operative, the heated products produced in the first receptacle 303, will pass through a second receptacle 307, in which a second substrate 309 is located, so as to produce an inhalable medium that contains one or more constituents derived from the first and second substrates 305, 309. A second heater 313 may also heat the second substrate 309. Alternatively, if the second flow path 361 is operative, the heated products produced in the first receptacle 303, will pass through a channel, or conduit 317 but not pass through the second receptacle 307, so as to produce an inhalable medium that contains one or more constituents derived from the first substrate 305 only.

Accordingly, the first flow path 351 therefore includes the first receptacle 303 and the second receptacle 307. The second flow path 361 includes the first receptacle 303 but not the second receptacle 307. The second flow path may include the first receptacle 303 and the conduit 317 arranged outside of the first flow path 351. In the example of Figure 5, the first flow path 351 includes the first outlet 321 and the second flow path 361 includes the second outlet 319. Each flow path therefore may have its own separate outlet.

In the example of Figure 5, a removable mouthpiece 323 may also be provided that can be attached to the apparatus 301, for example at each of the outlets 321, 319. Therefore, unlike in Figure 4, the second substrate 309 may remain in situ when the second flow path 361 is operative. The user can therefore move the removable mouthpiece 323 between outlets 321, 319 depending upon which flow path is, or is to be, operative.

In one specific example, the action of removing the mouthpiece 323 from an outlet may automatically cause the outlet to close, such that an open aperture is not visible. This can stop debris from entering, or exiting the flow path. Similarly, the action of affixing the mouthpiece to an outlet may automatically cause the outlet to open. In other examples, a user can manually open or close the outlets 321, 319 by removing or placing a stopper in the outlet, or by manually causing a guard to open or close or cover the outlet.

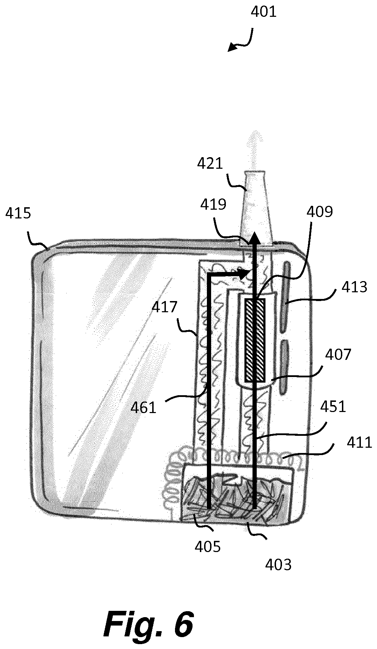

Figure 6 shows an alternative apparatus 401 for generating an inhalable medium. In broad outline, the apparatus 401 comprises a housing 415, that defines a first flow path (illustrated by arrow 451 and a second flow path (illustrated by arrow 461). In use, only one of these flow paths may be operative at any one time, and a user may select, or choose which is the operative flow path. In the example of Figure 6, the housing comprises a single outlet 419, and a mouthpiece 421 affixed to the outlet 419. The apparatus 401 depicts two separate flow paths that share a single outlet, and therefore join to allow the heated products to exit the single outlet.

Figure 6 depicts a first heater 411 within the apparatus 401 to heat a first substrate 405 in a first receptacle 403 to form heated products. If the first flow path 451 is operative, the heated products produced in the first receptacle 403, will pass through a second receptacle 407, in which a second substrate 409 is located, so as to produce an inhalable medium that contains one or more constituents derived from the first and second substrates 405, 409. A second heater 413 may also heat the second substrate 409. Alternatively, if the second flow path 461 is operative, the heated products produced in the first receptacle 403, will pass through a channel, or conduit 417 but not pass through the second receptacle 407, so as to produce an inhalable medium that contains one or more constituents derived from the first substrate 405 only.

Accordingly, the first flow path 451 therefore includes the first receptacle 403 and the second receptacle 407. The second flow path 461 includes the first receptacle 403 but not the second receptacle 407. The second flow path may include the first receptacle 403 and the conduit 417 arranged outside of the first flow path 451. In the example of Figure 6, the first flow path 451 includes the outlet 419, and the second flow path 461 also includes the outlet 419. The flow paths therefore share a single outlet, and the flow paths are arranged to facilitate this. The housing therefore comprises a single outlet shared by both the first and second flow paths. Such a construction may provide an apparatus that is simpler to use, requires less cleaning, and may be more compact than an apparatus with two or more outlets. In such an apparatus, the first and second flow paths would therefore join before the outlet. In the example of Figure 6, the conduit 417 is an inverted "L" shape, and joins the first flow path 451 before the outlet 419. Therefore a portion of the first and second flow paths share a common path.

Figures 7A and 7B show an alternative apparatus 501 for generating an inhalable medium. The apparatus 501 is substantially similar to the apparatus 301 of Figure 5, but further depicts means for selecting one or more flow paths. In broad outline, the apparatus 501 comprises a housing 515, that defines a first flow path (illustrated by arrow 551) and a second flow path (illustrated by arrow 561). Again, in use, only one of these flow paths may be operative at any one time, and an operative flow path can be selected using a selection assembly 523. The selection assembly 523 may comprise one or more parts, for example parts 525 and 527 (discussed in more detail below). In the example apparatus 501, the housing 115 comprises a first outlet 521 and a second outlet 519. This provides the user with simple and effective means to choose, or select between at least the first and second flow paths, as they desire.

Figures 7A and 7B depict a first heater 511 within the apparatus 501 to heat a first substrate 505 in a first receptacle 503 to form heated products. In Figure 7A, the first flow path 551 is operative, such that the heated products produced in the first receptacle 503, will pass through a second receptacle 507, in which a second substrate 509 is located, so as to produce an inhalable medium that contains one or more constituents derived from the first and second substrates 505, 509. A second heater 513 may also heat the second substrate 509. Alternatively, if the second flow path 561 is operative, as in Figure 7B, the heated products produced in the first receptacle 503, will pass through a channel, or conduit 517 but not pass through the second receptacle 507, so as to produce an inhalable medium that contains one or more constituents derived from the first substrate 505 only.

In the example apparatus 501, the first flow path 551 also includes the first outlet 521 and the second flow path 561 also includes the second outlet 519. Each flow path therefore may have its own separate outlet. Accordingly, should the user select the second flow path, the user would need to inhale the inhalable medium from the second outlet. Separate outlets can provide a simpler construction. An outlet may be an aperture defined by the housing, through which the inhalable medium passes out of the housing, before being inhaled by a user.

In the example apparatus 501, a removable mouthpiece may also be provided that can be attached to the apparatus 501, for example at each of the outlets 521, 519. The mouthpiece can therefore be moved between the first and second outlets to provide a simple way of preparing the flow paths for use and may provide a more lightweight, more compact construction in contrast to two separate mouthpieces. The removeable mouthpiece may affix to the housing by a screw fit, snap fit, or friction fit, for example.

As mentioned briefly above, the apparatus 501 comprises a flow path selection assembly 523 movable between at least a first position in which the first flow path 551 is operative (as in Figure 7A), and a second position in which the second flow path 561 is operative (as in Figure 7B). The flow path selection assembly may comprise one or more moving parts that open or close sections within the housing to allow the inhalable medium to flow along the operative flow path.

In some examples, the flow path selection assembly 523 comprises a control member 525, the control member 525 being actuatable by a user, wherein actuation of the control member causes the flow path selection assembly 523 to move between at least the first and second positions. In the example of Figures 7A and 7B, the flow path selection assembly further comprises a sliding, or slideable member 527, where the slideable member 527 is moved between a first location and a second location as a result of actuation of the control member 525. This provides the user with a means to easily select between at least the first and second flow paths. The control member may comprise, for example, one or more buttons, one or more switches, or a touch screen.

As can be seen in Figure 7A, the flow path selection assembly 523 is arranged according to a first position. An aperture formed in the slideable member 527 allows heated products produced by the first substrate 505 to pass along the first flow path 551 uninterrupted. In contrast, a solid portion of the slideable member 527 restricts heated products from flowing along the second flow path 561, such that the heated products cannot flow along the conduit 517.

To select the second flow path 561, the flow path selection assembly 523 can be rearranged into a second position. In the example of Figures 7A and 7B, a user may actuate the control member 525. For example, the control member may be a button, and pressing the button can cause the slideable member 527 to slide into the housing 515 such that the aperture within the slideable member 527 moves beneath the conduit 517 and a second solid portion of the slideable member covers the entrance leading towards the second receptacle 507. Figure 7B depicts the flow path selection assembly 523 arranged according to the second position. The control member 525 is shown depressed partially within the housing 515 (although in other examples the control member may be biased towards a single position regardless of which flow path is operative). Similarly, the slideable member 527 can be seen to have moved to another position within the housing. Therefore in Figure 7B, the aperture formed in the slideable member 527 allows heated products produced by the first substrate 505 to pass along the second flow path 561 uninterrupted. In contrast, the second solid portion of the slideable member 527 restricts heated products from flowing along the first flow path 551, such that the heated products cannot flow into the second receptacle 507.

Figures 7A and 7B depict just one example flow path selection assembly. Other flow path selection assemblies are envisaged, for example comprising fewer or more parts, or arranged differently within the housing.

In another alternative example, the action of removal or replacement of a removeable mouthpiece (not depicted in Figures 7A and 7B) onto the outlets 521, 519 may automatically cause the flow path selection assembly 523 to move between at least the first and second positions. As such, the mouthpiece may be a control member, or removal or replacement of the mouthpiece may cause a control member to be actuated directly or indirectly. This can provide a user-friendly method of switching flow paths. For example, the user would simply need to move the mouthpiece from the first outlet 521 to the second outlet 519 to select the second flow path 561 as the operative flow path.

In another alternative example, the second receptacle may be configured to receive a second substrate of the form depicted in Figure 3A (i.e. a consumable product configured to be partially inserted into the housing). The action of removal or replacement of the second substrate of this form out of, or into, the outlet, may automatically cause the flow path selection assembly to move between at least the first and second positions. As such, the second substrate may be a control member, or removal or replacement of the second substrate may cause a control member to be actuated directly or indirectly. This can provide a user-friendly method of switching flow paths. For example, the user would simply need to remove the second substrate from the first outlet to select the second flow path as the operative flow path.

In another alternative example, the control member may be a button or switch that causes the slideable member to be electronically moved, for example by one or more motors. The control member may be part of a user interface, for example on a touchscreen integrated into the apparatus.

In another alternative example, a plurality of flow diverters provided within the apparatus may open and close as a result of actuation of a control member. Example flow diverters may include valves. For example, in the example apparatus 401 of Figure 6, three valves may be present: a first valve arranged at the top of the first receptacle 403 to allow, or restrict heated products to flow along the first flow path 451, a second valve arranged at the top of the first receptacle 403 to allow, or restrict heated products to flow along the second flow path 461, and a third valve arranged at the tip of the inverted "L" shaped conduit, to stop the inhalable medium flowing into the second flow path 461 when the first flow path 451 is operative.

Although Figures 7A and 7B depict the second substrate as being fully received in the housing 515, it will be appreciated that the substrate may take the form of that depicted in Figure 3A. It should also be noted that although Figures 3-5 depict two flow paths, the example apparatus may comprise three or more flow paths. The selection assembly may therefore be moveable between three or more positions to facilitate selection of a flow path. Furthermore, it will be appreciated that the flow path selection assembly may also be incorporated into any of Figures 1-4.

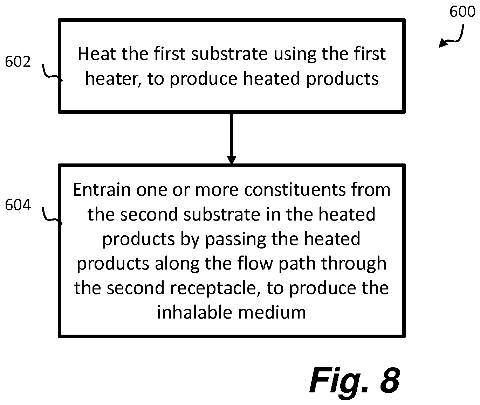

Referring to Figure 8, there will now be described an example of steps in a method 600 of generating an inhalable medium using an apparatus, the apparatus comprising a first receptacle containing a first substrate, wherein the first substrate is a solid or a gel, a second receptacle containing a second substrate, wherein the second substrate is a solid or a gel, a first heater arranged to heat the first substrate, a second heater arranged to heat the second substrate, and a housing defining a flow path including the first receptacle and the second receptacle.

In a first step 602, the method comprises heating the first substrate using the first heater, to produce heated products. In a second step 604, the method comprises entraining one or more constituents from the second substrate in the heated products by passing the heated products along the flow path through the second receptacle, to produce the inhalable medium.

The first and second substrates, when in solid form, may include materials that provide volatilised components upon heating, typically in the form of an aerosol. Suitable materials include any tobacco-containing material and may, for example, include one or more of tobacco per se, tobacco derivatives, expanded tobacco, reconstituted tobacco, ground tobacco, tobacco extract, homogenised tobacco or tobacco substitutes. In the case of tobacco, the material may be in the form of a rod of tobacco, a pod or plug of tobacco, loose tobacco, agglomerates, etc., and may be in relatively dry form or in relatively moist form for example. Suitable materials may include other, non-tobacco, products, which, depending on the product, may or may not contain nicotine. Non-tobacco products may include flavour pouches.

The first and second substrates, when in gel form, may include sheets of gel configured to provide volatilised components upon heating, typically in the form of an aerosol. Gel based substrates may or may not include nicotine.

In order to address various issues and advance the art, the entirety of this disclosure shows by way of illustration and example various embodiments in which the claimed invention may be practised and which provide for a superior apparatus arranged to generate an inhalable medium. The advantages and features of the disclosure are of a representative sample of embodiments only, and are not exhaustive and/or exclusive. They are presented only to assist in understanding and teach the claimed and otherwise disclosed features. It is to be understood that advantages, embodiments, examples, functions, features, structures and/or other aspects of the disclosure are not to be considered limitations on the disclosure as defined by the claims, and that other embodiments may be utilised and modifications may be made without departing from the scope of protection which is defined by the claims.

## Claims

1. Apparatus (300; 301; 401; 501) for generating an inhalable medium, the apparatus comprising:
a first receptacle (302; 303; 403; 503) configured to receive a first substrate (304; 305; 405; 505), wherein the first substrate is a solid or a gel;
a second receptacle (306; 307; 407; 507) configured to receive a second substrate (308; 309; 409; 509), wherein the second substrate is a solid or a gel;
a first heater (310; 311; 411; 511) arranged to heat the first substrate in use;
a second heater (312; 313; 413; 513) arranged to heat the second substrate in use; **characterized by**:
a housing defining:
a first flow path (350; 351; 451; 551) including the first receptacle and the second receptacle; and
a second flow path (360; 361; 461; 561) including the first receptacle and not the second receptacle;
wherein:
the first flow path is arranged such that in use heated products produced by heating the first substrate received in the first receptacle pass through the second receptacle to produce the inhalable medium; and
the second flow path is arranged such that in use heated products produced by the first substrate received in the first receptacle form the inhalable medium and pass along the second flow path without passing through the second receptacle.

2. An apparatus according to claim 1, wherein one of the first and second receptacles is configured to receive a consumable product of a predetermined size, the consumable product comprising one of the first and second substrates, and wherein the other of the first and second receptacles is configured to directly receive the other of the first and second substrates.

3. An apparatus according to claim 2, wherein a receptacle configured to directly receive a substrate comprises:
a chamber (120); and
a member (118) movable to cover or uncover an opening providing access to the chamber.

4. An apparatus according to any preceding claim, wherein the apparatus comprises a flow path selection assembly (523) movable between at least a first position in which the first flow path is operative, and a second position in which the second flow path is operative.

5. An apparatus according to claim 4, wherein the flow path selection assembly comprises a control member (525), the control member being actuatable by a user, wherein actuation of the control member causes the flow path selection assembly to move between at least the first and second positions.

6. An apparatus according to any one of claims 1-5, wherein the housing comprises a first outlet (320; 321; 521) and a second outlet (318; 319; 519), and wherein the first flow path includes the first outlet, and the second flow path includes the second outlet.

7. An apparatus according to claim 6, further comprising a removeable mouthpiece (323), the removeable mouthpiece attachable to both the first and second outlets.

8. An apparatus according to any one of claims 1-5, wherein the housing comprises an outlet (419), and wherein both the first flow path and the second flow path include the outlet.

9. A system comprising:
an apparatus according to any one of claims 1-8; and
at least one of the first and second substrates.

10. A method of generating an inhalable medium using an apparatus (300; 301; 401; 501), the apparatus comprising a first receptacle (302; 303; 403; 503) containing a first substrate (304; 305; 405; 505) wherein the first substrate is a solid or a gel, a second receptacle (306; 307; 407; 507) containing a second substrate (308; 309; 409; 509) wherein the second substrate is a solid or a gel, a first heater (310; 311; 411; 511) arranged to heat the first substrate, a second heater (312; 313; 413; 513) arranged to heat the second substrate, and a housing defining a first flow path (350; 351; 451; 551) including the first receptacle and the second receptacle and a second flow path (360; 361; 461; 561) including the first receptacle and not the second receptacle, the method comprising at least one of:
heating the first substrate using the first heater, to produce heated products and entraining one or more constituents from the second substrate in the heated products by passing the heated products along the first flow path through the second receptacle, to produce the inhalable medium;
heating the first substrate using the first heater, to produce the inhalable medium and passing the inhalable medium along the second flow path.

11. A method according to claim 10, further comprising:
receiving a consumable product of a predetermined size in one of the first and second receptacles, the consumable product comprising one of the first and second substrates; and
directly receiving the other of the first and second substrates in the other of the first and second receptacles.

12. A method according to claim 10, the method further comprising:
selecting one of the first and second flow paths as an operative flow path.

13. A method according to claim 12, wherein selecting one of the first and second flow paths comprises moving a selection assembly (523) between at least a first position, in which the first flow path is the operative flow path, and a second position in which the second flow path is the operative flow path.

14. A method according to any one of claims 10-13, the method further comprising at least one of:
passing the inhalable medium along the first flow path and through a first outlet (320; 321; 521) in the housing; and
passing the inhalable medium along the second flow path and through a second outlet (318; 319; 519) in the housing.

15. A method according to any one of claims 10-13, the method further comprising at least one of:
passing the inhalable medium along the first flow path and through an outlet (419) in the housing; and
passing the inhalable medium along the second flow path and through the outlet in the housing.

## Patentansprüche

1. Einrichtung (300; 301; 401; 501) zum Erzeugen eines inhalierbaren Mediums, wobei die Einrichtung Folgendes umfasst:
einen ersten Behälter (302; 303; 403; 503), der gestaltet ist, ein erstes Substrat (304; 305; 405; 505) aufzunehmen, wobei das erste Substrat ein Feststoff oder ein Gel ist;
einen zweiten Behälter (306; 307; 407; 507), der gestaltet ist, ein zweites Substrat (308; 309; 409; 509) aufzunehmen, wobei das zweite Substrat ein Feststoff oder ein Gel ist;
eine erste Heizvorrichtung (310; 311; 411; 511), die angeordnet ist, in Gebrauch das erste Substrat zu erhitzen;
eine zweite Heizvorrichtung (312; 313; 413; 513), die angeordnet ist, in Gebrauch das zweite Substrat zu erhitzen; **gekennzeichnet durch**:
ein Gehäuse, das Folgendes definiert:
einen ersten Strömungsweg (350; 351; 451; 551), der den ersten Behälter und den zweiten Behälter beinhaltet, und
einen zweiten Strömungsweg (360; 361; 461; 561), der den ersten Behälter und nicht den zweiten Behälter beinhaltet;
wobei:
der erste Strömungsweg so angeordnet ist, dass in Gebrauch erhitzte Produkte, die durch Erhitzen des ersten Substrats, das in dem ersten Behälter aufgenommen ist, erzeugt werden, durch den zweiten Behälter strömen, um das inhalierbare Medium zu erzeugen; und
der zweite Strömungsweg so angeordnet ist, dass in Gebrauch erhitzte Produkte, die durch das erste Substrat, das in dem ersten Behälter aufgenommen ist, erzeugt werden, das inhalierbare Medium bilden und entlang des zweiten Strömungswegs strömen, ohne durch den zweiten Behälter zu strömen.

2. Einrichtung nach Anspruch 1, wobei einer des ersten und zweiten Behälters gestaltet ist, ein Verbrauchsmaterial einer vorbestimmten Größe aufzunehmen, wobei das Verbrauchsmaterial eines des ersten und zweiten Substrats umfasst, und wobei der andere des ersten und zweiten Behälters gestaltet ist, das andere des ersten und zweiten Substrats aufzunehmen.

3. Einrichtung nach Anspruch 2, wobei ein Behälter, der gestaltet ist, direkt ein Substrat aufzunehmen, umfasst:
eine Kammer (120); und
ein Element (118), das beweglich ist, um eine Öffnung, die Zugang zu der Kammer bereitstellt, zu bedecken oder aufzudecken.

4. Einrichtung nach einem vorstehenden Anspruch, wobei die Einrichtung eine Strömungswegauswahlbaugruppe (523) umfasst, die zwischen mindestens einer ersten Position, in der der erste Strömungsweg betriebsbereit ist, und einer zweiten Position, in der der zweite Strömungsweg betriebsbereit ist, beweglich ist.

5. Einrichtung nach Anspruch 4, wobei die Strömungswegauswahlbaugruppe ein Steuerelement (525) umfasst, wobei das Steuerelement von einem Benutzer betätigbar ist, wobei Betätigung des Steuerelements bewirkt, dass die Strömungswegauswahlbaugruppe sich zwischen mindestens der ersten und der zweiten Position bewegt.

6. Einrichtung nach einem der Ansprüche 1-5, wobei das Gehäuse einen ersten Auslass (320; 321; 521) und einen zweiten Auslass (318; 319; 519) umfasst und wobei der erste Strömungsweg den ersten Auslass beinhaltet und der zweite Strömungsweg den zweiten Auslass beinhaltet.

7. Einrichtung nach Anspruch 6, weiter umfassend ein entfernbares Mundstück (323), wobei das entfernbare Mundstück sowohl an dem ersten als auch zweiten Auslass befestigbar ist.

8. Einrichtung nach einem der Ansprüche 1-5, wobei das Gehäuse einen Auslass (419) umfasst und wobei sowohl der erste Strömungsweg als auch der zweite Strömungsweg den Auslass beinhalten.

9. System, umfassend:
eine Einrichtung nach einem der Ansprüche 1-8; und
mindestens eines des ersten und zweiten Substrats.

10. Verfahren zum Erzeugen eines inhalierbaren Mediums unter Verwendung einer Einrichtung (300; 301; 401; 501), wobei die Einrichtung einen ersten Behälter (302; 303; 403; 503), der ein erstes Substrat (304; 305; 405; 505) enthält, wobei das erste Substrat ein Feststoff oder ein Gel ist, einen zweiten Behälter (306; 307; 407; 507), der ein zweites Substrat (308; 309; 409; 509) enthält, wobei das zweite Substrat ein Feststoff oder ein Gel ist, eine erste Heizvorrichtung (310; 311; 411; 511), die angeordnet ist, das erste Substrat zu erhitzen, eine zweite Heizvorrichtung (312; 313; 413; 513), die angeordnet ist, das zweite Substrat zu erhitzen, und ein Gehäuse umfasst, das einen ersten Strömungsweg (350; 351; 451; 551), der den ersten Behälter und den zweiten Behälter beinhaltet, und einen zweiten Strömungsweg (360; 361; 461; 561), der den ersten Behälter und nicht den zweiten Behälter beinhaltet, definiert, wobei das Verfahren mindestens eines umfasst von:
Erhitzen des ersten Substrats unter Verwendung der ersten Heizvorrichtung, um erhitzte Produkte zu erzeugen, und Mitführen eines oder mehrere Bestandteile von dem zweiten Substrat in den erhitzten Produkten, durch Strömen erhitzter Produkte entlang des ersten Strömungswegs durch den zweiten Behälter, um das inhalierbare Medium zu erzeugen;
Erhitzen des ersten Substrats unter Verwendung der ersten Heizvorrichtung, um das inhalierbare Medium zu erzeugen, und Strömen des inhalierbaren Mediums entlang des zweiten Strömungswegs.

11. Verfahren nach Anspruch 10, weiter umfassend:
Aufnehmen eines Verbrauchsmaterials einer vorbestimmten Größe in einem des ersten und zweiten Behälters, wobei das Verbrauchsmaterial eines des ersten und zweiten Substrats umfasst; und
direktes Aufnehmen des anderen des ersten und zweiten Substrats in dem anderen des ersten und zweiten Behälters.

12. Verfahren nach Anspruch 10, wobei das Verfahren weiter umfasst:
Auswählen eines des ersten und zweiten Strömungswegs als einen betriebsbereiten Strömungsweg.

13. Verfahren nach Anspruch 12, wobei Auswählen eines des ersten und zweiten Strömungswegs Bewegen einer Auswahlbaugruppe (523) zwischen mindestens einer ersten Position, in der der erste Strömungsweg der betriebsbereite Strömungsweg ist, und einer zweiten Position, in der der zweite Strömungsweg der betriebsbereite Strömungsweg ist, umfasst.

14. Verfahren nach einem der Ansprüche 10-13, wobei das Verfahren weiter mindestens eines umfasst von:
Strömen des inhalierbaren Mediums entlang des ersten Strömungswegs und durch einen ersten Auslass (320; 321; 521) in dem Gehäuse; und
Strömen des inhalierbaren Mediums entlang des zweiten Strömungswegs und durch einen zweiten Auslass (318; 319; 519) in dem Gehäuse.

15. Verfahren nach einem der Ansprüche 10-13, wobei das Verfahren weiter mindestens eines umfasst von:
Strömen des inhalierbaren Mediums entlang des ersten Strömungswegs und durch einen Auslass (419) in dem Gehäuse; und
Strömen des inhalierbaren Mediums entlang des zweiten Strömungswegs und durch den Auslass in dem Gehäuse.

## Revendications

1. Appareil (300; 301; 401; 501) pour générer un milieu inhalable, l'appareil comprenant :
un premier réceptacle (302 ; 303 ; 403 ; 503) configuré pour recevoir un premier substrat (304 ; 305 ; 405 ; 505), dans lequel le premier substrat est un solide ou un gel ;
un second réceptacle (306 ; 307 ; 407 ; 507) configuré pour recevoir un second substrat (308 ; 309 ; 409 ; 509), dans lequel le second substrat est un solide ou un gel ;
un premier dispositif de chauffage (310 ; 311 ; 411 ; 511) agencé pour chauffer le premier substrat en cours d'utilisation ;
un second dispositif de chauffage (312 ; 313 ; 413 ; 513) agencé pour chauffer le second substrat en cours d'utilisation ; **caractérisé par** :
un boîtier définissant :
un premier chemin d'écoulement (350; 351; 451; 551) incluant le premier réceptacle et le second réceptacle ; et
un second chemin d'écoulement (360; 361; 461; 561) incluant le premier réceptacle et non le second réceptacle ;
dans lequel :
le premier chemin d'écoulement est agencé de sorte qu'en cours d'utilisation les produits chauffés produits par le chauffage du premier substrat reçu dans le premier réceptacle passent à travers le second réceptacle pour produire le milieu inhalable ; et
le second chemin d'écoulement est agencé de sorte qu'en cours d'utilisation les produits chauffés produits par le premier substrat reçu dans le premier réceptacle forment le milieu inhalable et passent le long du second chemin d'écoulement sans passer à travers le second réceptacle.

2. Appareil selon la revendication 1, dans lequel l'un parmi les premier et second réceptacles est configuré pour recevoir un produit consommable d'une taille prédéterminée, le produit consommable comprenant l'un parmi les premier et second substrats, et dans lequel l'autre parmi les premier et second réceptacles est configuré pour recevoir directement l'autre parmi les premier et second substrats.

3. Appareil selon la revendication 2, dans lequel un réceptacle configuré pour recevoir directement un substrat comprend :
une chambre (120) ; et
un élément (118) mobile pour couvrir ou découvrir une ouverture fournissant un accès à la chambre.

4. Appareil selon l'une quelconque des revendications précédentes, dans lequel l'appareil comprend un ensemble de sélection de chemin d'écoulement (523) mobile entre au moins une première position dans laquelle le premier chemin d'écoulement est opérationnel, et une seconde position dans laquelle le second chemin d'écoulement est opérationnel.

5. Appareil selon la revendication 4, dans lequel l'ensemble de sélection de chemin d'écoulement comprend un élément de commande (525), l'élément de commande pouvant être actionné par un utilisateur, dans lequel l'actionnement de l'élément de commande amène l'ensemble de sélection de chemin d'écoulement à se déplacer entre au moins les première et seconde positions.

6. Appareil selon l'une quelconque des revendications 1-5, dans lequel le boîtier comprend une première sortie (320 ; 321 ; 521) et une seconde sortie (318 ; 319 ; 519), et dans lequel le premier chemin d'écoulement inclut la première sortie, et le second chemin d'écoulement inclut la seconde sortie.

7. Appareil selon la revendication 6, comprenant en outre un embout buccal amovible (323), l'embout buccal amovible pouvant être attaché à la fois aux première et seconde sorties.

8. Appareil selon l'une quelconque des revendications 1-5, dans lequel le boîtier comprend une sortie (419), et dans lequel à la fois le premier chemin d'écoulement et le second chemin d'écoulement incluent la sortie.

9. Système comprenant :
un appareil selon l'une quelconque des revendications 1-8 ; et
au moins l'un pami les premier et second substrats.

10. Procédé de génération d'un milieu inhalable utilisant un appareil (300 ; 301 ; 401 ; 501), l'appareil comprenant un premier réceptacle (302 ; 303 ; 403 ; 503) contenant un premier substrat (304 ; 305 ; 405 ; 505) dans lequel le premier substrat est un solide ou un gel, un second réceptacle (306 ; 307 ; 407 ; 507) contenant un second substrat (308 ; 309; 409; 509) dans lequel le second substrat est un solide ou un gel, un premier dispositif de chauffage (310 ; 311 ; 411 ; 511) agencé pour chauffer le premier substrat, un second dispositif de chauffage (312 ; 313 ; 413 ; 513) agencé pour chauffer le second substrat, et un boîtier définissant un premier chemin d'écoulement (350 ; 351 ; 451 ; 551) incluant le premier réceptacle et le second réceptacle et un second chemin d'écoulement (360 ; 361; 461 ; 561) incluant le premier réceptacle et non le second réceptacle, le procédé comprenant au moins l'une des étapes suivantes :
le chauffage du premier substrat en utilisant le premier dispositif de chauffage, pour produire des produits chauffés et l'entraînement d'un ou plusieurs constituants provenant du second substrat dans les produits chauffés en faisant passer les produits chauffés le long du premier chemin d'écoulement à travers le second réceptacle, pour produire le milieu inhalable ;
le chauffage du premier substrat en utilisant le premier dispositif de chauffage, pour produire le milieu inhalable et le passage du milieu inhalable le long du second chemin d'écoulement.

11. Procédé selon la revendication 10, comprenant en outre :
la réception d'un produit consommable d'une taille prédéterminée dans l'un parmi les premier et second réceptacles, le produit consommable comprenant l'un parmi les premier et second substrats ; et
la réception directe de l'autre parmi les premier et second substrats dans l'autre parmi les premier et second réceptacles.

12. Procédé selon la revendication 10, le procédé comprenant en outre :
la sélection d'un parmi les premier et second chemins d'écoulement comme chemin d'écoulement opérationnel.

13. Procédé selon la revendication 12, dans lequel la sélection d'un parmi les premier et second chemins d'écoulement comprend le déplacement d'un ensemble de sélection (523) entre au moins une première position, dans laquelle le premier chemin d'écoulement est le chemin d'écoulement opérationnel, et une seconde position dans laquelle le second chemin d'écoulement est le chemin d'écoulement opérationnel.

14. Procédé selon l'une quelconque des revendications 10-13, le procédé comprenant en outre au moins l'une des étapes suivantes :
le passage du milieu inhalable le long du premier chemin d'écoulement et à travers une première sortie (320 ; 321 ; 521) dans le boîtier ; et
le passage du milieu inhalable le long du second chemin d'écoulement et à travers une seconde sortie (318 ; 319 ; 519) dans le boîtier.

15. Procédé selon l'une quelconque des revendications 10-13, le procédé comprenant en outre au moins l'une des étapes suivantes :
le passage du milieu inhalable le long du premier chemin d'écoulement et à travers une sortie (419) dans le boîtier ; et
le passage du milieu inhalable le long du second chemin d'écoulement et à travers la sortie dans le boîtier.
